# EUROPÄISCHE PATENTSCHRIFT

(11) **EP 1 201 225 B1**
(45) Veröffentlichungstag und Bekanntmachung des Hinweises auf die Patenterteilung: **16.04.2003**
(21) Anmeldenummer: 01123313.7
(22) Anmeldetag: 08.10.2001
(51) Int. Cl.: A61K 7/13

(54) **Haarfärbemittel**
Hair dye composition
Composition de coloration capillaire

(30) Priorität: 14.10.2000 DE 10051039
(43) Veröffentlichungstag der Anmeldung: 02.05.2002
(73) Patentinhaber: KPSS-Kao Professional Salon Services GmbH, 64297 Darmstadt (DE)
(72) Erfinder: Lorenz, Heribert, 64401 Gross Bieberau (DE); Kaffenberger, Klaus, 64665 Alsbach-Hähnlein (DE)

(56) Entgegenhaltungen:
- EP-A- 0 402 163
- EP-A- 0 467 026
- DE-A- 4 122 748

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können und das Haar selbst bei kurzfristiger wiederholter Anwendung nicht schädigt.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht werden können.

Es wurde auch bereits vorgeschlagen, diese Lücke durch Verwendung alternativer Entwicklersubstanzen zu schließen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann Abstriche in der Farbintensität anderer Nuancen hingenommen werden.

Eine weitgehend optimale Lösung dieses Problems wird durch den in der EP-A 615 743 beschriebenen Einsatz von 2-(2'-Hydroxyethylamino)-5-aminotoluol bzw. dessen wasserlöslichen Salzen und die aus der EP-B 467 026 bekannten Triaminohydroxypyrimidine, insbesondere 2,5,6-Triamino-4-hydroxypyrimidin, 2,4,5-Triamino-6-hydroxypyrimidin, 4,5,6-Triamino-2-hydroxypyrimidin bzw. deren Salze, insbesondere die Sulfate, als Entwicklersubstanzen in Haarfärbemitteln erreicht. Selbst dadurch bleiben jedoch noch farbtechnische Wünsche offen.

Die Erfindung geht daher von der Aufgabenstellung aus, ein Haarfärbemittel zu schaffen, das zur Herstellung einer großen Anzahl von Farbtönen geeignet ist und das Haar selbst bei kurzzeitiger wiederholter Anwendung nicht schädigt.

Diese Aufgabe wird dadurch gelöst, daß ein solches Haarfärbemittel ein mit Peroxid reagierendes Oxidationsfarbstoff-System enthält, das aus einer Kombination aus 1,3-Dimethyl-3,4,5,6-tetrahydro-2-(1H)-pyrimidinon und mindestens einer weiteren Kuppler- und/oder Entwicklersubstanz besteht

Diese ist vorzugsweise ausgewählt aus der Gruppe 1-Methoxy-2-amino-4-(β-hydroxyethylamino)benzol, 2-Amino-3-hydroxypyridin, 2,5-Diaminopyridin, 2,6-Diaminopyridin, 3-Amino-2-methylamino-6-methoxypyridin, 2-(Dimethylamino)-5-aminopyridin, 1-(β-Hydroxyethyl)-2,5-diamino-benzol, 2-(2'-Hydroxyethylamino)-5-aminotoluol, 1-Amino-4-bis-(2'-hydroxyethyl)aminobenzol, 2,4,5,6-Tetraminopyrimidin, 2,5,6-Triamino-4-hydroxypyrimidin, 2-Amino-5-N,N-diethylaminotoluol, 1,4-Diaminobenzol, 1,3-Diaminobenzol, 2,5-Diaminotoluol, Resorcin, 2-Methylresorcin, 4-Chlorresorcin, 2-Amino-4-chlorphenol, 1,6-Dihydroxynaphthalin, 2-Aminophenol, 3-Aminophenol,1-Methyl-2-hydroxy-4-aminobenzol, 5-Amino-2-methoxyphenol, 2-Methyl-5-hydroxyethylaminophenol, 4-Amino-3-methylphenol, 5-Amino-2-methylphenol, 2-Amino-4-β-hydroxyethylaminoanisol bzw. deren wasserlösliche Salze und/oder 1-Naphthol.

Damit soll jedoch der Zusatz weiterer Entwickler- und Kupplersubstanzen keineswegs ausgeschlossen sein.

Bei Anwendung dieser Zusammensetzungen auf Basis einer üblichen Grundlage werden nach der Oxidation mit Peroxid sehr ausdrucksvolle, intensive, dauerhafte Haarfärbungen vor allem im Gelb-, Gold-, Orange- und Braunbereich erhalten, die durch Zusatz entsprechender weiterer Entwickler- und Kupplersubstanzen noch zu anderen Farbnuancen variiert werden können.

Auch die zusätzliche Mitverwendung weiterer, an sich bekannter Entwicklersubstanzen ist möglich. Neben den bereits oben genannten sind hierbei insbesondere noch 5-Aminosalicylsäure und/oder 1,2,4-Triaminobenzol zu erwähnen.

Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Das bevorzugte Gewichtsverhältnis von 1,3-Dimethyl-3,4,5,6-tetrahydro-2-(1H)-pyrimidinon zu den weiteren Entwickler- und Kupplersubstanzen liegt dabei zwischen etwa 1 : 8 bis 8 : 1, vorzugsweise etwa 1:5 bis 5 : 1, insbesondere 1 : 2 bis 2 : 1.

Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | @ 100,00 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

| Beispiel 1: | |
|---|---|
| 0,58 (Gew.-%) | 1,3-Dimethyl-3,4,5,6-Tetrahydro-2-(1H)-pyrimidinon (PC 94) |
| 0,91 | p-Toluylendiaminsulfat |

### Färbung:

Intensives Orangebraun.

| Beispiel 2: | |
|---|---|
| 0,58 (Gew.-%) | PC 94 |
| 0,55 | 1-Methyl-2-hydroxy-4-aminobenzol |

### Färbung:

Intensives Hellgelborange.

| Beispiel 3: | |
|---|---|
| 0,58 (Gew.-%) | PC94 |
| 0,49 | p-Phenylendiamin |

### Färbung:

Intensives Braunviolett.

| Beispiel 4: | |
|---|---|
| 0,58 (Gew.-%) | PC 94 |
| 1,07 | 2,4,5,6-Tetraaminopyrimidinsulfat |

### Färbung:

Intensives Hellgelb.

| Beispiel 5: | |
|---|---|
| 0,58 (Gew.-%) | PC 94 |
| 1,08 | 4-Hydroxy-2,5,6-triaminopyrimidinsulfat |

### Färbung:

Glänzendes Hellgelb.

| Beispiel 6: | |
|---|---|
| 0,58 (Gew.-%) | PC 94 |
| 1,42 | 2-(2'-Hydroxyethylamino)-5-aminotoluolsulfat |

### Färbung:

Intensives Orangebraun.

| Beispiel 7: | |
|---|---|
| 0,58 (Gew.-%) | PC 94 |
| 0,55 | 4-Amino-3-methylphenol |

### Färbung:

Glänzendes Gelborange.

| Beispiel 8: | |
|---|---|
| 0,58 (Gew.-%) | PC 94 |
| 0,50 | 2-Amino-3-hydroxypyridin |

### Färbung:

Intensives glänzendes Mittelgelborange.

| Beispiel 9: | |
|---|---|
| 0,58 (Gew.-%) | PC 94 |
| 1,98 | p-Toluylendiaminsulfat |
| 0,55 | 1-Methyl-2-hydroxy-4-aminobenzol |

### Färbung:

Intensives Violettpurpur.

## Patentansprüche

1. Haarfärbemittel auf Basis eines mit Peroxid reagierenden Oxidationsfarbstoff-Vorprodukts, enthaltend 1,3-Dimethyl-3,4,5,6-tetrahydro-2-(1H)-pyrimidinon und mindestens eine weitere Entwickler- undloder Kupplersubstanz.

2. Haarfärbemittel nach Anspruch 1, enthaltend als weitere Entwickler- und Kupplersubstanz(en) mindestens eine Komponente, ausgewählt aus der Gruppe 1-Methoxy-2-amino-4-(β-hydroxyethylamino)benzol, 2-Amino-3-hydroxypyridin, 2,5-Diaminopyridin, 2,6-Diaminopyridin, 3-Amino-2-methylamino-6-methoxypyridin, 2-(Dimethylamino)- 5-aminopyridin, 1-(β-Hydroxyethyl)-2,5-diaminobenzol, 2-(2'-Hydroxyethylamino)-5-aminotoluol, 1-Amino-4-bis-(2'-hydroxyethyl)aminobenzol, 2,4,5,6-Tetraamino-pyrimidin, 2,5,6-Triamino-4-hydroxypyrimidin, 2-Amino-5-N,N-diethylaminotoluol, 1,4-Diaminobenzol, 1,3-Diaminobenzol, 2,5-Diaminotoluol, Resorcin, 2-Methylresorcin, 4-Chlorresorcin, 2-Amino-4-chlorphenol, 1,6-Dihydroxynaphthalin, 2-Aminophenol, 3-Aminophenol,1-Methyl-2-hydroxy-4-aminobenzol, 5-Amino-2-methoxyphenol, 2-Methyl-5-hydroxyethylaminophenol, 4-Amino-3-methylphenol, 5-Amino-2-methylphenol, 2-Amino-4-β-hydroxyethylaminoanisol bzw. deren wasserlösliche Salze und/oder 1-Naphthol.

## Claims

1. Composition for the dyeing of human hair on the basis of an oxidation dyestuff precursor reacting with peroxide, comprising 1.3-dimethyl-3,4,5,6-tetrahydro-2-(1H)-pyrimidinone and at least one additional developing and/or coupling substance.

2. Hair dyeing composition according to claim 1, comprising as additional developing and coupling substance at least one component selected from the group 1-methoxy-2-amino-4-(β-hydroxyethyl amino)benzene, 2-amino-3-hydroxypyridine, 2.5-diaminopyridine, 2.6-diaminopyridine, 3-amino-2-methyl amino-6-methoxypyridine, 2-(dimethyl amino)-5-aminopyridine, 1-(β-hydroxyethyl)-2.5-diaminobenzene, 2-(2'-hydroxyethyl amino)-5-aminotoluene, 1-amino-4-bis-(2'-hydroxyethyl) aminobenzene, 2.4.5.6-tetramino-pyrimidine, 2.5.6-triamino-4-hydroxy pyrimidine, 2-amino-5-N,N-diethyl amino-toluene, 1.4-diaminobenzene, 1.3-diaminobenzene, 2.5-diaminotoluene, resorcinol, 2-methyl resorcinol, 4-chlororesorcinol, 2-amino-4-chlorophenol, 1.6-dihydroxynaphthaline, 2-aminophenol, 3-aminophenol,1-methyl-2-hydroxy-4-aminobenzene, 5-amino-2-methoxyphenol, 2-methyl-5-hydroxyethyl amino-phenol, 4-amino-3-methyl phenol, 5-amino-2-methyl phenol, 1-naphthol and/or 2-amino-4-β-hydroxyethyl aminoanisol or the water-soluble salts thereof.

## Revendications

1. Agent de coloration des cheveux à base d'un précurseur de colorant d'oxydation qui réagit avec un peroxyde, contenant de la 1,3-diméthyl-3,4,5-tétrahydro-2-(1H)-pyrimidinon et au moins une autre substance de développement et/ou de couplage.

2. Agent de coloration des cheveux selon la revendication 1, contenant comme autre substance ou comme autres substances de développement et de couplage au moins un composant sélectionné dans le groupe constitué du 1-méthoxy-2-amino-4-(β-hydroxyéthylamino)benzène, de la 2-amino-3-hycroxypyridine, de la 2,5-diaminopyridine, de la 2,6-diaminopyridine, de la 3-amino-2-méthylamino-6-méthoxypyridine, de la 2-(diméthylamino)-5-aminopyridine, du 1-(β-hydroxyéthyl)-2,5-diamino-benzène, du 2-(2'-hydroxyéthylamino)-5-aminotoluène, du 1-amino-4-bis-(2'-hydroxyéthyl)aminobenzène, de la 2,4,5,6-tétraaminopyridmidine, de la 2,5,6-triamino-4-hydroxypyrimidine, du 2-amino-5-N,N-diéthylaminotoluène, du 1,4-diaminobenzène, du 1,3-diaminobenzène, du 2,5-diaminotoluène, de la résorcine, de la 2-méthylrésorcine, de la 4-chlororésorcine, du 2-amino-4-chlorophénol, de la 1,6-dihydroxynaphtaline, du 2-aminophénol, du 3-aminophéncl, du 1-méthyl-2-hydroxy-4-aminobenzène, du 5-amino-2-méthoxyphénol, du 2-méthyl-5-hydroxyéthylaminophénol, du 4-amino-3-méthylphénol, du 5-amino-2-méthylphénol, du 2-amino-4-β-hydroxyéthylaminoanisol ou de leurs sels solubles dans l'eau, et/ou du 1-naphtène.
